# EUROPÄISCHE PATENTSCHRIFT

(11) **EP 1 337 246 B1**
(45) Veröffentlichungstag und Bekanntmachung des Hinweises auf die Patenterteilung: **22.03.2006**
(21) Anmeldenummer: 01984780.5
(22) Anmeldetag: 28.11.2001
(51) Int. Cl.: A61K 31/00, A61P 13/10

(54) **VERWENDUNG VON 6-DIMETHYLAMINOMETHYL-1-PHENYL-CYCLOHEXANVERBINDUNGEN ZUR THERAPIE DER HARNINKONTINENZ**
USE OF 6-DIMETHYLAMINOMETHYL-1-PHENYL-CYCLOHEXANE COMPOUNDS FOR TREATING URINARY INCONTINENCE
UTILISATION DE COMPOSES 6-DIMETHYLAMINOMETHYLE-1-PHENYL-CYCLOHEXANE POUR TRAITER L'INCONTINENCE URINAIRE

(30) Priorität: 30.11.2000 DE 10059411
(43) Veröffentlichungstag der Anmeldung: 27.08.2003
(73) Patentinhaber: Grünenthal GmbH, 52078 Aachen (DE)
(72) Erfinder: CHRISTOPH, Thomas, 52080 Aachen (DE); FRIDERICHS, Elmar, 52223 Stolberg (DE)
(86) Internationale Anmeldenummer: PCT/EP2001/013909
(87) Internationale Veröffentlichungsnummer: WO 2002/043712

(56) Entgegenhaltungen:
- EP-A- 1 005 861
- US-A- 5 733 936

## Beschreibung

Die Erfindung betrifft die Verwendung von 6-Dimethylaminomethyl-1-phenyl-cyclohexanverbindungen als freie Basen und/oder in Form physiologisch verträglicher Salze zur Herstellung eines Arzneimittels zur Behandlung von vermehrtem Hamdrang bzw. Haminkontinenz sowie entsprechende Arzneimittel und Verfahren zur Behandlung von vermehrtem Hamdrang bzw. Haminkontinenz.

Haminkontinenz ist der unwillkürliche Hamabgang. Dieser tritt unkontrolliert auf, wenn der Druck innerhalb der Harnblase den Druck übersteigt, der zum Schließen des Hamleiters notwendig ist. Ursachen können zum einen ein erhöhter interner Blasendruck (z. B. durch Detrusorinstabilität) mit der Folge der Dranginkontinenz und zum anderen ein erniedrigter Sphinkterdruck (z. B. nach Geburt oder chirurgischen Eingriffen) mit der Folge der Streßinkontinenz sein. Der Detrusor ist die grob gebündelte mehrschichtige Blasenwandmuskulatur, deren Kontraktion zur Hamentleerung führt, der Sphinkter der Schließmuskel der Harnröhre. Es treten Mischformen dieser Inkontinenzarten sowie die sogenannte Überflußinkontinenz (z B. bei benigner Prostatahyperplasie) oder Reflexinkontinenz (z. B. nach Rückenmarksschädigungen) auf. Näheres dazu findet sich bei Chutka, D. S. und Takahashi, P. Y., 1998, Drugs 560: 587-595.

Hamdrang ist der auf Harnentleerung (Miktion) abzielende Zustand vermehrter Blasenmuskelspannung bei Annäherung an die Blasenkapazität (bzw. bei deren Überschreitung). Dabei wirkt diese Anspannung als Miktionsreiz. Unter einem vermehrten Hamdrang versteht man dabei insbesondere das Auftreten vorzeitigen oder gehäuften manchmal sogar schmerzhaften Hamdrangs bis hin zum sog. Harnzwang. Das führt in der Folge zu einer deutlich häufigeren Miktion. Ursachen können u.a. Harnblasenentzündungen und neurogene Blasenstörungen sowie auch Blasentuberkulose sein. Es sind aber noch nicht alle Ursachen geklärt.

Vermehrter Harndrang wie auch Harninkontinenz werden als extrem unangenehm empfunden und es besteht ein deutlicher Bedarf bei von diesen Indikationen betroffenen Personen, eine möglichst langfristige Verbesserung zu erreichen.

Üblicherweise werden vermehrter Hamdrang und insbesondere Harninkontinenz medikamentös mit Substanzen behandelt, die an den Reflexen des unteren Hamtraktes beteiligt sind (Wein, A. J., 1998, Urology 51 (Suppl. 21): 43-47). Meistens sind dies Medikamente, die eine hemmende Wirkung auf den Detrusormuskel, der für den inneren Blasendruck verantwortlich ist, haben. Diese Medikamente sind z. B. Parasympatholytika wie Oxybutynin, Propiverin oder Tolterodin, trizyklische Antidepressiva wie Imipramin oder Muskelrelaxantien wie Flavoxat. Andere Medikamente, die insbesondere den Widerstand der Hamröhre oder des Blasenhalses erhöhen, zeigen Affinitäten zu α-Adrenorezeptoren wie Ephedrin, zu β-Adrenorezeptoren wie Clenbutarol oder sind Hormone wie Östradiol. Auch bestimmte Opioide, Diarylmethylpiperazine und -piperidine, sind für diese Indikation in der WO 93/15062 beschrieben. Für Tramadol wurde ein positiver Effekt auf die Blasenfunktion in einem Rattenmodell rhythmischer Blasenkontraktionen nachgewiesen (Nippon-Shinyaku, WO 98/46216).

Bei den hier in Frage kommenden indikationen ist zu beachten, daß es sich im allgemeinen um sehr langfristige medikamentöse Anwendungen handelt und sich die Betroffenen im Gegensatz zu vielen Situationen, in denen Analgetika eingesetzt werden, einer sehr unangenehmen, aber nicht unaushaltbaren Situation gegenüber sehen. Daher ist hier - noch mehr als bei Analgetika - darauf zu achten, Nebenwirkungen zu vermeiden, will der Betroffene nicht ein Übel gegen das andere tauschen. Auch sind bei einer dauerhaften Haminkontinenzbehandlung auch analgetische Wirkungen weitgehend unerwünscht.

Aufgabe der vorliegenden Erfindung war es daher, Stoffe aufzufinden, die zur Behandlung von vermehrtem Hamdrang bzw. Haminkontinenz hilfreich sind und bei den wirksamen Dosen bevorzugt gleichzeitig geringere Nebenwirkungen und/oder analgetische Wirkungen zeigen als aus dem Stand der Technik bekannt.

Überraschenderweise wurde nun gefunden, daß Verbindungen gemäß allgemeiner Formel I eine hervorragende Wirkung auf die Blasenfunktion besitzen und demzufolge gut zur Behandlung entsprechender Erkrankungen geeignet sind.

Dementsprechend ist Erfindungsgegenstand die Verwendung einer 6-Dimethylaminomethyl-1-phenyl-cyclohexanverbindung gemäß allgemeiner Formel I 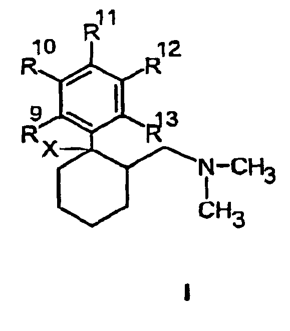 , worin
X ausgewählt ist aus OH, F, Cl, H oder OC(O)CH₃ und
R⁹ bis R¹³ , wobei 3 oder 4 der Reste R⁹ bis R¹³ H entsprechen müssen, jeweils unabhängig voneinander ausgewählt sind aus
H, Cl, F, OH, CF₂H, CF₃ oder unsubstituiertes C₁₋₄-Alkyl, verzweigt oder unverzweigt; OR¹⁴ oder SR¹⁴, mit R¹⁴ ausgewählt aus unsubstituiertem C₁₋₃-Alkyl, verzweigt oder unverzweigt;
oder
R⁹ und R¹⁰ oder R¹⁰ und R¹¹ zusammen einen OCH₂O-, OCH₂CH₂O-, OCH=CH-, CH=CHO-, CH=C(CH3)O-. OC(CH3)=CH-, (CH₂)₄- oder OCH=CHO-Ring bilden,
in Form ihrer Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren
zur Herstellung eines Arzneimittels zur Behandlung von vermehrtem Harndrang bzw. Haminkontinenz.

Überraschenderweise hatte sich herausgestellt, daß die genannten Substanzen einen physlologische Parameter, der bei vermehrtem Harndrang bzw. Haminkontinenz von Bedeutung sind, deutlich positiv beeinflußen, nämlich das Interkontraktionsintervall, bzw. die Verringerung der rhythmischen Blasenkontraktionen. Diese Veränderung kann eine deutliche Erleichterung im symptomatischen Bild von betroffener Patienten bedeuten. Entsprechende Verbindungen und deren Herstellung sind aus der DE 195 25 137 A1 bekannt.

Sie übertreffen in ihrer Wirksamkeit für diese indikation noch deutlich das bekannte Tramadol, das aber nicht unter diese Erfindung fällt, so daß hier insbesondere die Verwendung einer Verbindung gemäß Formel I ausgenommen ist, bei der X OH entspricht, wenn R⁹, R¹¹ und R¹³ H entsprechen und einer von R¹⁰ oder R¹² H und der andere OCH₃ entspricht.

Im Sinne dieser Erfindung versteht man unter Alkyl-Resten gesättigte und ungesättigte, verzweigte und unverzweigte Kohlenvwasserstoffe, die auch mindestens einfach substituiert sein können. Bevorzugte Alkyl-Reste sind Methyl. Ethyl, Vinyl (Ethenyl), Propyl, Allyl (2-Propenyl), 1-Propinyl, Methylethyl, n-Butyl, sek.-Butyl, tert.-Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, CHF₂, CF₃ oder CH₂OH.

Weiter versteht man unter Cykloalkyl-Resten im Sinne dieser Erfindung gesättigte cyklische Kohlenwasserstoffe, die auch mindestens einfach substituiert sein können. Bevorzugte Cykloalkyl-Reste sind Cyclopropyl, Cyclobutyl, Cyclopentyl, Cyclohexyl oder Cycloheptyl.

Dabei versteht man im Zusammenhang mit Alkyl und Cycloalkyl unter dem Begriff substituiert im Sinne dieser Erfindung die Substitution eines Wasserstoffrestes durch F, Cl, Br, I, NH₂, SH oder OH, wobei unter "mehrfach substituiert" zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂.

Dabei versteht man im Zusammenhang mit Phenyl, Benzyl oder Phenethyl unter substituiert bevorzugt die Substitution mit H, F, Cl, Br, I, CH₂F, CHF₂, CF₃, OH, SH, OR¹⁹, OCF₃, SR¹⁹, NH₂, CONH₂, SOCH₃, SOCF₃, SO₂CH₃, SO₂CF₃, CN, COOR¹⁹, NO₂; C₁₋₆-Alkyl, verzweigt oder unverzweigt, gesättigt oder ungesättigt, unsubstituiert oder ein- oder mehrfach substituiert; Phenyl, unsubstituiert;
mit R¹⁹ ausgewählt aus C₁₋₆-Alkyl; verzweigt oder unverzweigt, gesättigt oder ungesättigt, unsubstituiert oder ein- oder mehrfach substituiert: oder C₃₋₇-Cycloalkyl.

Geeignete Salze im Sinne dieser Erfindung und in jeder der beanspruchten Verwendungen sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Bevorzugt ist dabei die Verwendung von Verbindungen gemäß Formel I worin X ausgewählt ist aus
OH, F oder H.

Bevorzugt ist weiter die Verwendung von Verbindungen gemäß Formel I worin R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃, OCH₃ oder SCH₃
oder R¹² und R¹¹ einen 3,4-OCH=CH-Ring bilden
insbesondere worin.
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise OH, CF₂H, OR¹⁴ oder SCH₃, insbesondere OH oder OC₁₋₃₋Alkyl, vorzugsweise OH oder OCH₃,
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht
oder,
wenn R⁹, R¹⁰, R¹² und R¹³ H entsprechen. R¹¹ ausgewählt ist aus CF₃, CF₂H, Cl oder F, vorzugsweise F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

Weiter bevorzugt ist dabei auch die Verwendung von Verbindungen gemäß Formel I, worin
R⁹, R¹¹ und R¹³ H entsprechen, einer von R¹⁰ oder R¹² auch H entspricht, während der andere ausgewählt ist aus:
Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

Es ist auch bevorzugt, wenn Verbindungen gemäß Formel I in Form ihrer Diastereomeren mit der relativen Konfiguration Ia 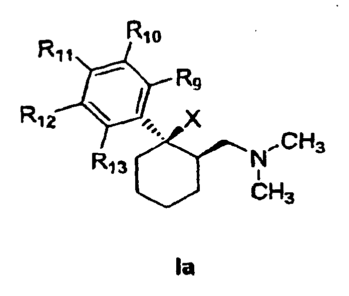 vorliegen, insbesondere in Mischungen mit höherem Anteil dieses Diastereomeren im Vergleich zum anderen Diastereomeren oder als reines Diastereomer verwendet werden.

Weiter ist es bevorzugt, wenn die Verbindungen der Formel I in Form des (+)-Enantiomeren, insbesondere in Mischungen mit höherem Anteil des (+)-Enantiomeren im Vergleich zum (-)-Enantlomeren einer racemischen Verbindung oder als reines (+)-Enantiomer verwendet werden.

Generell ist auch bei bevorzugter Verwendung des (+)-Enantiomeren ein gegenüber dem (+)-Enantiomeren geringerer Anteil an (-)-Enantiomer akzeptabel und darf - muß aber nicht - bei der erfindungsgemässen Verwendung enthalten sein.

Besonders bevorzugt ist die Verwendung einer Verbindung ausgewählt aus folgender Gruppe:
■ (+)-(1R,2R)-3-(2-Dimethylaminomethyl-1-fluoro-cyclohexyl)-phenol,
■ (+)-(1S,2R)-3-(2-Dimethylaminomethyl-cyclohexyl)-phenol oder
■ (-)-(1R,2S)-3-(2-Dimethylaminomethyl-cyclohexyl)-phenol,
vorzugsweise als Hydrochlorid.

Auch wenn die erfindungsgemässen Verwendungen lediglich geringe Nebenwirkungen zeigen, kann es beispielsweise zur Vermeidung von bestimmten Formen der Abhängigkeit auch von Vorteil sein, neben Verbindungen gemäß allgemeiner Formel I auch Morphinantagonisten, insbesondere Naloxon, Naltrexon und/oder Levallorphan, zu verwenden.

Geeignete Salze im Sinne dieser Erfindung und in den beanspruchten Arzneimitteln sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Geeignete Zusatz- und/oder Hilfsstoffe im Sinne dieser Erfindung sind alle dem Fachmann aus dem Stand der Technik bekannten Stoffe zur Er reichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen können die erfindungsgemäßen Verbindungen verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muß, besonders bevorzugt.

Weiter bevorzugt sind Arzneimittel, die wenigstens 0.05 bis 90,0 % des Wirkstoffes enthalten, insbesondere niedrige wirksame Dosierungen, um Neben- oder analgetische Wirkungen zu vermeiden. Üblicherweise werden 0,1 bis 5000 mg/kg. insbesondere 1 bis 500 mg/kg, vorzugsweise 2 bis 250 mg/kg Körpergewicht wenigstens einer Verbindung der Formel I appliziert. Ebenso bevorzugt und üblich ist aber auch die Applikation von 0,01 - 5 mg/kg, vorzugsweise 0.03 bis 2 mg/kg, insbesondere 0,05 bis 1 mg/kg Körpergewicht.

Hilfsstoffe können beispielsweise sein: Wasser. Ethanol, 2-Propanol. Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Cellulaseacetat, Schellack, Cetylalkohol, Polyvinylpyrrolidon, Paraffine, Wachse, natürliche und synthetische Gummis. Akaziengummi, Alginate, Dextran, gesättigte und ungesättigte Fettsäuren, Stearinsäure, Magnesiumstearat, Zinkstearat. Glycerylstearat, Natriumlaurylsulfat, genießbare Öle, Sesamöl, Kokusnußöl, Erdnußöl, Sojabohnenöl, Lecithin. Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid. Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid. Talkum, Kaolin, Pectin, Crospovidon, Agar und Bentonit.

Die Herstellung der efindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur I oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzungzu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Es ist weiter bevorzugt, wenn in den erfindungsgemäßen Arzneimitteln Verbindungen gemäß allgemeiner Formel I enthalten sind, die in Form ihrer Diastereomeren mit der relativen Konfiguration Ia 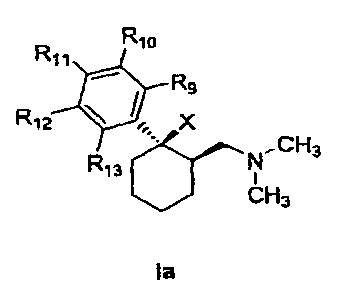 vorliegen, insbesondere in Mischungen mit höherem Anteil dieses Diastereomeren im Vergleich zum anderen Diastereomeren oder als reines Diastereomer.

Es ist weiter bevorzugt, wenn in den erfindungsgemäßen Arzneimitteln Verbindungen gemäß allgemeiner Formel I enthalten sind, die in Form des (+)-Enantiomeren vorliegen, insbesondere in Mischungen mit höherem Anteil des (+)-Enantiomeren im Vergleich zum (-)-Enantiomeren einer racemischen Verbindung oder als reines (+)-Enantiomer.

Generell ist auch bei bevorzugter Verwendung des (+)-Enantiomeren ein gegenüber dem (+)-Enantiomeren geringerer Anteil an (-)-Enantiomer akzeptabel und darf - muß aber nicht - bei den erfindungsgemässen Arzneimitteln enthalten sein.

Besonders bevorzugt sind erfindungsgemäße Arzneimittel, die mindestens eine Verbindung ausgewählt aus folgender Gruppe enthalten:
■ (+)-(1R,2R)-3-(2-Dimethylaminomethyl-1-fluoro-cyclohexyl)-phenol,
■ (+)-(1S,2S)-3-(2-Dimethylaminomethyl-cyclohexyl)-phenol oder
■ (-)-(1R,2R)-3-(2-Dimethylaminomethyl-cyclohexyl)-phenol,
vorzugsweise als Hydrochlorid.

Weiter betrifft die Erfindung auch ein Verfahren zur Behandlung von vermehrtem Harndrang bzw. Haminkontinenz, bei dem die 6-Dimethytaminomethyl-1-phenyl-cyclohexanverbindungen gemäß allgemeiner Formel I in Form ihrer Razemate; Enantiomere, Diastereomere, insbesondere Mischungen ihrer Enantiomere oder Diastereomere oder eines einzelnen Enantiomers oder Diastereomers; als freie Base und/oder in Form physiologisch verträglicher Salze verwendet werden.

Die folgenden Beispiele sollen die Erfindung erläutern, ohne daß der Gegenstand der Erfindung darauf beschränkt wäre.

### Beispiele

### Beispiel 1: Liste der getesteten Substanzen:

Es folgt eine Liste der auf Ihre Wirksamkeit getesteten Verbindungen:

| **Name** | **Verbdg. Nr.** |
|---|---|
| (+)-(1R,2R)-3-(2-Dimethylaminomethyl-1-fluoro-cyclohexyl)-pheno, Hydrochlorid | **18** |
| (+)-(1S,2S)-3-(2-Dimethylaminomethyl-cyclohexyl)-phenol, Hydrochlorid | **19** |
| (-)-(1R,2R)-3-(2-Dimethylaminomethyl-cyclohexyl)-phenol, Hydrochlorid | **20** |
| *rac*-Tramadol | **23** |

### Beispiel 2. Testsystem Cystometrie an der narkotisierten naiven Ratte

Die cystometrische Untersuchung an naiven weiblichen Ratten wurde nach der Methode von Kimura et al. (Kimura et al., 1996, Int. J. Urol. 3:218-227) durchgeführt. An narkotisierten, ventilierten Ratten wird das Abdomen eröffnet und die Harnleiter abgebunden. Der Harn wird von den Nieren abgeleitet. Ein Katheter wird in die Blase eingeführt und fixiert. Über diesen wird Saline mittels Infusionspumpe in die Blase infundiert, bis diese rhythmische Spontanaktivität in Form von Kontraktionen zeigt, welche über einen angeschlossenen Druckaufnehmer aufgenommen werden können. Die Testsubstanz wird nach Erreichen stabiler Ausgangswerte in kumulativer Weise i.v. appliziert. Eine Beeinflussung der Blasenfunktion äußert sich über die Unterdrückung der Spontankontraktionen. Dabei gilt als Parameter für die Unterdrückung das Ausbleiben der Kontraktionen über einen Zeitraum von 10 min.

Bei allen hier aufgelisteten Substanzen war eine Unterdrückung der Spontankontraktionen in den Ratten meßbar, wobei Tabelle 1 den Mittelwert der niedrigsten Dosis aus mindestens 2 Versuchen angibt, bei der erstmals Kontraktionen über einen Zeitraum von 10 min ausbleiben.

**Tabelle 1:(n entspricht der Anzahl der in den Wert eingegangenen Versuche)**

| **Verbdg.-Nr.** | **Niedrigste Dosis (mg/kg)** |
|---|---|
| **18** | 0,2 (n=3) |
| **19** | 0,1 (n=3) |
| **20** | 0,5 (n=3) |
| **23 (Tramadol)** | 5,3 (n=3) |

Die untersuchten Substanzen zeigen eine positive Wirkung auf die Blasenregulation und sind somit geeignet zur Behandlung der Haminkontinenz und erscheinen darin auch gegenüber Tramadol überlegen.

### Beispiel 3: Parenterale Applikationsform

1 g der Verbindung 19 wird in 11 Wasser für Injektionszwecke bei Raumtemperatur gelöst und anschließend durch Zugabe von NaCl auf isotone Bedingungen eingestellt.

## Patentansprüche

1. Verwendung einer 6-Dimethylaminomethyl-1-phenylcyclohexanverbindung gemäß allgemeiner Formel I 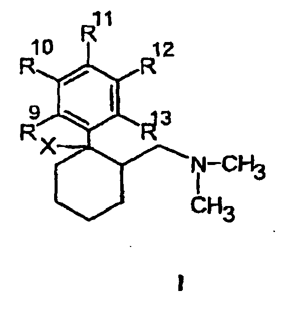 , worin
X ausgewählt ist aus OH, F, Cl, H oder OC(O)CH₃ und
R⁹ bis R¹³, wobei 3 oder 4 der Reste R⁹ bis R¹³ H entsprechen müssen, jeweils unabhängig voneinander ausgewählt sind aus
H, Cl, F, OH, CF₂H, CF₃ oder unsubstituiertes C₁₋₄-Alkyl, verzweigt oder unverzweigt; OR¹⁴ oder SR¹⁴, mit R¹⁴ ausgewählt aus unsubstituiertes C₁₋₃-Alkyl, verzweigt oder unverzweigt;
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
mit der Maßgabe, daß, wenn R⁹, R¹¹ und R¹³ H entsprechen, und einer von R¹⁰ oder R¹² H und der andere OCH₃ entspricht, X nicht OH sein darf,
in Form ihrer Razemate; Enantiomere, Diastereomere, Mischungen ihrer Enantiomere oder Diastereomere oder eines einzelnen Enantiomers oder Diastereomers; ihrer Basen und/oder Salze physiologisch verträglicher Säuren
zur Herstellung eines Arzneimittels zur Behandlung von vermehrtem Hamdrang bzw. Haminkontinenz.

2. Verwendung gemäß Anspruch 1, **dadurch gekennzeichnet, daß** X ausgewählt ist aus OH, F oder H.

3. Verwendung gemäß einem der Ansprüche 1 oder 2, **dadurch gekennzeichnet, daß** R⁹ bis R¹³ unabhängig voneinander ausgewählt sind aus H, Cl, F, OH, CF₂H, CF₃, OCH₃ oder SCH₃
oder R¹² und R¹¹ einen 3.4-OCH=CH-Ring bilden.

4. Verwendung gemäß einem der Ansprüche 1 bis 3. **dadurch gekennzeichnet, daß**,
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴,
oder,
wenn R⁹ und R¹³ H entsprechen und R¹¹ OH, OCH₃, Cl oder F entspricht, einer von R¹⁰ oder R¹² auch H entspricht, während der andere OH, OCH₃, Cl oder F, entspricht,
oder,
wenn R⁹, R¹⁰, R¹² und R¹³ H entsprechen, R¹¹ ausgewählt ist aus CF₃, CF₂H, Cl oder F.
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

5. Verwendung gemäß einem der Ansprüche 1 bis 3, **dadurch gekennzeichnet, daß**,
wenn R⁹, R¹¹ und R¹³ H entsprechen, einer von R¹⁰ oder R¹² auch H entspricht, während der andere ausgewählt ist aus SCH₃ oder OCH₃.

6. Verwendung gemäß einem der Ansprüche 1 bis 5, **dadurch gekennzeichnet, daß** Verbindungen der Formel I in Form ihrer Diastereomeren mit der relativen Konfiguration Ia 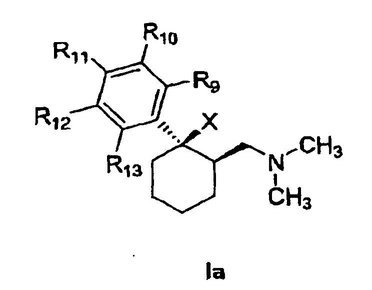 vorliegen, in Mischungen mit höherem Anteil dieses Diastereomeren im Vergleich zum anderen Diastereomeren oder als reines Diastereomer verwendet werden.

7. Verwendung gemäß einem der Ansprüche 1 bis 5, **dadurch gekennzeichnet, daß** die Verbindungen der Formel I in Form des (+)-Enantiomeren, in Mischungen mit höherem Anteil des (+)-Enantiomeren im Vergleich zum (-)-Enantiomeren einer racemischen Verbindung oder als reines (+)-Enantiomer verwendet werden.

8. Verwendung gemäß einem der Ansprüche 1 bis 7, **dadurch gekennzeichnet, daß** eine Verbindung ausgewählt aus folgender Gruppe verwendet wird:
■ (+)-(1R,2R)-3-(2-Dimethylaminomethyl-1-fluoro-cyclohexyl)-phenol,
■ (+)-(1S,2S)-3-(2-Dimethylaminomethyl-cyclohexyl)-phenol oder
■ (-)-(1R,2R)-3-(2-Dimethylaminomethyl-cyclohexyl)-phenol,
respektive als Hydrochlorid.

## Claims

1. Use of a 6-dimethylaminomethyl-1-phenyl-cyclohexane compound according to general formula I 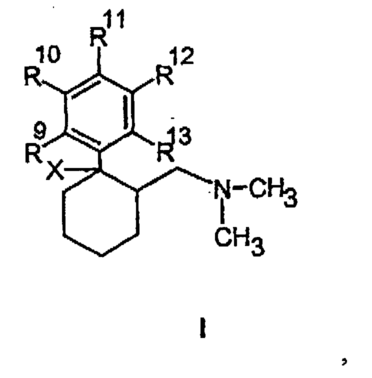 wherein
X is chosen from OH, F, Cl, H or OC(O)CH₃, and
R⁹ to R¹³, where 3 or 4 of the radicals R⁹ to R¹³ must correspond to H, in each case independently of one another are chosen from
H, Cl, F, OH, CF₂H, CF₃ or unsubstituted C₁₋₄₋alkyl, branched or unbranched; OR¹⁴ or SR¹⁴, where R¹⁴ is chosen from unsubstituted C₁₋₃-alkyl, branched or unbranched;
or
R⁹ and R¹⁰ or R¹⁰ and R¹¹ together form an OCH₂O, OCH₂CH₂O, OCH=CH, CH=CHO, CH=C(CH3)O, OC(CH3)=CH, (CH₂)₄ or OCH=CHO ring,
with the proviso that if R⁹, R¹¹ and R¹³ correspond to H and one of R¹⁰ or R¹² corresponds to H and the other corresponds to OCH₃, X may not be OH
in the form of their racemates; enantiomers, diastereomers, mixtures of their enantiomers or diastereomers, or of an individual enantiomer or diastereomer; their bases and/or salts of physiologically acceptable acids
for the preparation of a medicament for treatment of an increased urge to urinate or urinary incontinence.

2. Use according to claim 1, **characterized in that** X is chosen from OH, F or H.

3. Use according to one of claims 1 or 2, **characterized in that** R⁹ to R¹³ independently of one another are chosen from H, Cl, F, OH, CF₂H, CF₃, OCH₃ or SCH₃
or R¹² and R¹¹ form a 3,4-OCH=CH ring.

4. Use according to one of claims 1 to 3, **characterized in that**
if R⁹, R¹¹ and R¹³ correspond to H, one of R¹⁰ or R¹² also corresponds to H, while the other is chosen from:
Cl, F, OH, CF₂H, CF₃, OR¹⁴ or SR¹⁴,
or
if R⁹ and R¹³ correspond to H and R¹¹ corresponds to OH, OCH₃, Cl or F, one of R¹⁰ or R¹² also corresponds to H, while the other corresponds to OH, OCH₃, Cl or F,
or
if R⁹, R¹⁰, R¹² and R¹³ correspond to H, R¹¹ is chosen from CF₃, CF₂H, Cl or F,
or
if R¹⁰, R¹¹ and R¹² correspond to H, one of R⁹ or R¹³ also corresponds to H, while the other is chosen from OH, OC₂H₅ or OC₃H₇.

5. Use according to one of claims 1 to 3, **characterized in that**
if R⁹, R¹¹ and R¹³ correspond to H, one of R¹⁰ or R¹² also corresponds to H, while the other is chosen from SCH₃ or OCH₃,

6. Use according to one of claims 1 to 5, **characterized in that** compounds of the formula I are present in the form of their diastereomers with the relative configuration Ia 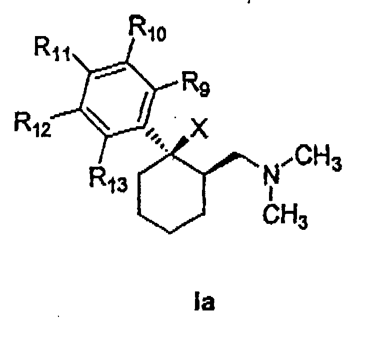 are used in mixtures with a higher content of this diastereomer compared with the other diastereomer or as the pure diastereomer.

7. Use according to one of claims 1 to 5, **characterized in that** the compounds of the formula I are used in the form of the (+)-enantiomer, in mixtures with a higher content of the (+)-enantiomer compared with the (-)-enantiomer of a racemic compound or as the pure (+)-enantiomer.

8. Use according to one of claims 1 to 7, **characterized in that** a compound chosen from the following group is used:
• (+)-(1R,2R)-3-(2-dimethylaminomethyl-1-fluoro-cyclohexyl)-phenol,
• (+)-(1S,2S)-3-(2-dimethylaminomethyl-cyclohexyl)-phenol
or
• (-)-(1R,2R)-3-(2-dimethylaminomethyl-cyclohexyl)-phenol,
respectively as the hydrochloride.

## Revendications

1. Utilisation d'un composé de 6-diméthylaminométhyl-1-phénylcyclohexane répondant à la formule générale I 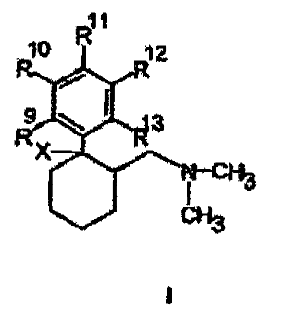 dans laquelle
X est choisi entre OH, F, Cl, H ou OC(O) CH₃ et
R⁹ à R¹³, dont trois ou quatre d'entre eux doivent correspondre à H, sont choisis, indépendamment les uns des autres, dans chaque cas entre
H, Cl, F, OH, CF₂H, CF₃ ou un reste alkyle en C₁ à C₄ non substitué, ramifié ou non ramifié ; OR¹⁴ ou SR¹⁴, R¹⁴ étant choisi entre des radicaux alkyle en C₁ à C₃ non substitués, ramifiés ou non ramifiés ;
ou bien
R⁹et R¹⁰ ou R¹⁰ et R¹¹ s'associent pour former un noyau OCH₂O-, OCH₂CH₂O-, OCH=CH-, CH=CHO-, CH=C(CH3)O-, OC(CH3)=CH-, (CH₂)₄- ou OCH=CHO-,
dans la mesure où, lorsque R⁹, R¹¹ et R¹³ correspondent à H, et l'un de R¹⁰ ou R¹² correspond à H et l'autre à OCH₃, X ne doit pas représenter OH,
sous forme de leurs racémates ; de leurs énantiomères, de leurs diastéréoisomères, de mélanges de leurs énantiomères ou diastéréoisomères ou d'un énantiomère ou diastéréoisomère individuel ; de leurs bases et/ou de leurs sels d'acides acceptables du point de vue physiologique,
pour la préparation d'un médicament destiné au traitement de la répétition du besoin impérieux d'uriner ou de l'incontinence d'urine.

2. Utilisation suivant la revendication 1, **caractérisée en ce que** X est choisi entre OH, F ou H.

3. Utilisation suivant l'une des revendications 1 ou 2, **caractérisée en ce que** R⁹ à R¹³ sont choisis, indépendamment les uns des autres, entre H, Cl, F, OH, CF₂H, CF₃, OCH₃ ou SCH₃
ou bien r¹² et r¹¹ forment ensemble un noyau 3,4-OCH=CH.

4. Utilisation suivant l'une des revendications 1 à 3, **caractérisée en ce que**
lorsque R⁹, R¹¹ et R¹³ correspondent à H, l'un de R¹⁰ ou R¹² correspond aussi à H tandis que l'autre est choisi entre :
Cl, F, OH, CF₂H, CF₃, OR¹⁴ ou SR¹⁴,
ou bien,
lorsque R⁹ et R¹³ correspondent à H et R¹¹ correspond à OH, OCH₃, Cl ou F, l'un de R¹⁰ ou R¹² correspond aussi à H tandis que l'autre correspond à OH, OCH₃, Cl ou F,
ou bien,
lorsque R⁹, R¹⁰, R¹² et R¹³ correspondent à H, R¹¹ est choisi entre CF₃, CF₂H, Cl ou F,
ou bien,
lorsque R¹⁰, R¹¹ et R¹² correspondent à H, l'un de R⁹
ou R¹³ correspond aussi à H tandis que l'autre est choisi entre OH, OC₂H₅ ou OC₃H₇.

5. Utilisation suivant l'une des revendications 1 à 3, **caractérisée en ce que**
lorsque R⁹, R¹¹ et R¹³ correspondent à H, l'un de R¹⁰ ou R¹² correspond aussi à H tandis que l'autre est choisi entre SCH₃ ou OCH₃.

6. Utilisation suivant l'une des revendications 1 à 5, **caractérisée en ce qu'**on utilise des composés de formule I présents sous forme de leurs diastéréoisomères ayant la configuration relative Ia 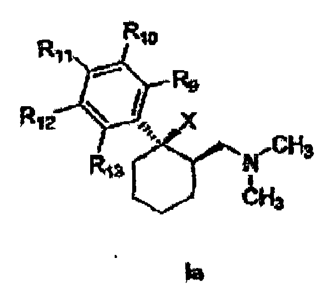 dans des mélanges contenant une plus forte proportion de ce diastéréoisomère comparativement à l'autre diastéréoisomère ou sous forme du diastéréoisomère pur.

7. Utilisation suivant l'une des revendications 1 à 5, **caractérisée en ce qu'**on utilise des composés de formule I sous forme du (+)-énantiomère dans des mélanges contenant une plus forte proportion du (+) -énantiomère comparativement au (-)-énantiomère d'un composé racémique ou sous forme du (+)-énantiomère pur.

8. Utilisation suivant l'une des revendications 1 à 7, **caractérisée en ce qu'**on utilise un composé choisi dans le groupe suivant :
• (+)-(1R,2R)-3-(2-diméthylaminométhyl-1-fluoro-cyclohexyl)-phénol,
• (+)-(1S,2S)-3-(2-diméthylaminométhyl-cyclohexyl)-phénol ou
• (-)-(1R,2R)-3-(2-diméthylaminométhyl-cyclohexyl)-phénol,
respectivement sous forme du chlorhydrate.
